# EUROPEAN PATENT SPECIFICATION

(11) **EP 4 655 022 B1**
(45) Date of publication and mention of the grant of the patent: **14.10.2026**
(21) Application number: 23820968.8
(22) Date of filing: 13.12.2023
(51) Int. Cl.: A61M 5/148, A61M 5/142, A61M 5/168, A61M 5/00, A61M 5/145, A61M 5/158, A61M 5/14

(54) **MEDICAMENT DELIVERY SYSTEM WITH A PUMP UNIT AND WITH AT LEAST ONE CASSETTE**
MEDIKAMENTENVERABREICHUNGSSYSTEM MIT EINER PUMPENEINHEIT UND MIT MINDESTENS EINER KASSETTE
SYSTÈME D'ADMINISTRATION DE MÉDICAMENT AVEC UNE UNITÉ DE POMPE ET AVEC AU MOINS UNE CARTOUCHE

(30) Priority: 26.01.2023 US 202363441208 P; 23.03.2023 EP 23163821
(43) Date of publication of application: 03.12.2025
(73) Proprietor: SHL Medical AG, 6302 Zug (CH)
(72) Inventor: SÄLL, Daniel, 131 28 Nacka Strand (SE); MYRMAN, Mattias, 131 28 Nacka Strand (SE); NEBY, Torbjörn, 131 28 Nacka Strand (SE)
(86) International application number: PCT/EP2023/085592
(87) International publication number: WO 2024/156433

(56) References cited:
- US-A1- 2006 015 084
- US-A1- 2021 154 401
- US-A1- 2021 369 958

## Description

### TECHNICAL FIELD

The present disclosure relates to a medicament delivery system and in particular to a medicament delivery system comprising a pump unit and at least one cassette separably attached to the pump unit.

### BACKGROUND

Medicament delivery devices for providing a plurality of different drugs to a patient are known in the state of the art. However, portable automated or semi-automated medicament delivery devices providing medication with a plurality of drugs are often bulky and complex to handle. In some systems, the patient has to connect each prescribed drug to a drive unit for medicament delivery. This can be difficult and time-consuming for medications consisting of multiple drugs that are required to be delivered in a certain order. Moreover, in such cases, error-proneness of connecting an undesired or even harmful drug to the drive unit may be high. In case a plurality of drug containers is stacked and connected to a single drive unit, control of delivery of a single drug and visual inspection may be challenging. If the drug is inspected from the side of the bag, however, wrinkles and welding lines may prevent the inspection. The drug may be deteriorated or contain particles. These drugs are often dosed according to a patient specific prescription and filled at a specialty pharmacy. It is possible that the pharmacy stacks the drugs in the wrong order which leads to a wrong injection sequence if the device cannot identify the drug position in the stack. Also, with a stacked solution it is difficult to digitally identify the different cassettes without being close with an identification module such as an RFID reader. This leads to a large device as it needs to be designed to identify up to four cassettes. However, this in turn makes the device unnecessary bulky for patients only requiring one drug. In addition, one area of concern is the possibility to match the wrong infusion set to the wrong drug. The needle needs to be matched with the drug cassette regarding needle gauge and needle penetration depth to ensure correct deposition of drug in the patient's tissue.

Moreover, a fluid connection between a drive unit and a plurality of medicament units may be difficult to seal and it may be complex to control delivery of only one of a plurality of attached medicament containers.

The present invention has been made in view of the above problems.

US 2021/154401 A1 discloses a device for the administration of liquids, drugs or nutrients to a patient. US 2021/369958 A1 discloses a pooling device for single or multiple medical containers. US 2006/015084 A1 discloses a liquid dispenser.

### SUMMARY

According to an aspect of the invention, inspection of a medicament inside a cassette or container is facilitated.

According to an aspect of the invention, erroneous medicament delivery is prevented.

According to an aspect of the invention, the structure and assembly of a medicament delivery system comprising a plurality of medicament cassettes is facilitated.

According to an aspect of the invention, effectively pressurising an interior of a medicament cassette is ensured.

According to an aspect of the invention, use of a medicament delivery system comprising a plurality of medicaments is simplified.

According to an aspect of the invention, a rigid and compact medicament delivery system is provided, independent of it being used with a single medicament cassettes or multiple medicament cassettes.

The invention is specified by the independent claims. Preferred embodiments are defined in the dependent claims. In the following description, although numerous features may be designated as optional, it is nevertheless acknowledged that all features comprised in the independent claims are not to be read as optional.

The present disclosure relates to a medicament delivery system comprising at least one cassette, wherein the cassette comprises a medicament bag provided in an interior of the cassette and an air inlet in fluid communication with the interior of the cassette. A pump unit comprises an air pump, a valve and an air outlet. Each cassette comprises cassette attachment means, and the pump unit comprises corresponding pump unit attachment means, wherein the pump unit is configured to be separably attached to an outer circumference of at least one cassette. The air outlet and the air inlet are fluidly connected after attachment of the at least one cassette with the pump unit. The air pump is configured to pressurise the interior of the cassette via the connected air outlet and the air inlet. The at least one cassette comprises a connection interface configured to be engageable with the cassette attachment means of another cassette.

Various embodiments may preferably implement the following features.

Preferably, the valve is configured to selectively direct an air flow from the pump to one of the at least one cassettes.

Preferably, the pump unit comprises a pressure sensor configured to measure a pressure of the interior of the cassette.

Preferably, the air pump comprises a pressure sensor configured to measure a pressure of the interior of the cassette.

Preferably, the at least one cassette is attached to the pump unit by a sliding movement.

Preferably, the at least one cassette comprises at least one guide protrusion as attachment means and the pump unit comprises at least one corresponding guide recess as attachment means.

Preferably, the pump unit comprises at least one guide protrusion as attachment means and the at least one cassette comprises at least one corresponding guide recess as attachment means.

Preferably, the at least one cassette is separably attached to the pump unit via the guide protrusion and the guide recess.

Preferably, the guide protrusion is formed in a protruding T-shape and the guide recess is formed in a corresponding recessed T-shape.

Preferably, four cassettes are attached to each other by the connection interface and the cassette attachment means.

Preferably, the four cassettes are configured to be arranged in a 90-degree angle with respect to each other or in a substantially rectangular shape.

Preferably, the system further comprises at least one adapter comprising adapter connection means configured to engage the connection interface of at least one cassette or pump unit attachment means.

Preferably, the adapter further comprises a connection interface.

Preferably, the adapter is configured to connect two cassettes, a cassette and an adapter, or two adapters with each other.

Preferably, four elements comprising cassettes and/or adapters are connected to form a substantially rectangular shape.

Preferably, cassettes and/or adapters are connected to each other such that they surround the pump unit.

Preferably, the valve is an x-way valve, wherein x equals the number of air outlets plus two.

Preferably, the valve is a six-way valve.

Preferably, the air inlet is disposed at a lower end of the cassette and the air outlet is disposed at a lower end of the pump unit.

Preferably, the air inlet protrudes from the cassette in a lateral and vertical direction having an L-shape.

Preferably, the air outlet is provided in a bottom surface of the pump unit.

Preferably, the air outlet and/or the air inlet comprises a sealing member configured to seal the connection between the air outlet and the air inlet in an air-tight manner when the at least one cassette is attached to the pump unit.

Preferably, the pump unit comprises four air outlets.

Preferably, an opening is provided in a bottom surface of the cassette and an outlet port of the medicament bag is provided in the opening.

Preferably, the pump unit comprises a switch and/or an indicator, wherein the switch and/or indicator is/are provided in a top surface opposite the bottom surface.

Preferably, the at least one cassette comprises an identification tag.

Preferably, the pump unit comprises at least one identification reader configured to read the identification tag.

Preferably, the system further comprises an infusion set for each cassette and each medicament bag comprises an outlet port configured to receive the infusion set.

Preferably, the infusion set comprises an identification tag and the pump unit comprises at least one identification reader configured to read the identification tag.

Preferably, the identification tag is an RFID tag.

Preferably, the pump unit comprises a processing unit configured to control at least one of the air pump, the valve, the indicator or the identification antenna.

Preferably, the switch is connected to the processing unit.

Preferably, the at least one cassette is formed of a transparent material.

Herein, the terms "top", "upper", "bottom" and "lower" refer to a vertical direction as shown in the figures (z-axis) while the terms "lateral" and "side" refer to a horizontal direction as shown in the figures (x-axis, left-right). The z-axis is perpendicular to a plane spanned by the x- and y-axis.

Further, the terms "longitudinal", "longitudinally", "axially" and "axial" refer to a direction extending in the direction of the longest extension of the device and/or component (vertical direction, z-axis).

Similarly, the terms "transverse", "transversal" and "transversally" refer to a direction generally perpendicular to the longitudinal direction (x-/y-axis).

Further, the terms "circumference", "circumferential", or "circumferentially" refer to a circumference or a circumferential direction relative to an axis of the device and/or component. Similarly, "radial" or "radially" refer to a direction extending radially relative to the axis, and "rotation", "rotational" and "rotationally" refer to rotation relative to the axis.

The term "separable" in accordance with the present invention means that the pump unit can be physically separated from the cassette(s). A separable pump unit can also be carried on or affixed to the cassette(s).

The term "independent" in accordance with certain embodiments of the present invention means that the pump unit is functionally independent from the cassette(s) and from the medicament bag(s). The pump unit according to the present invention can thus co-operate with a plurality of different cassettes, as desired by the user, wherein the cassette(s) is/are adapted to communicate with a pump unit. Thus, the user is provided with several options as regards the use of the pump unit. If desired, however, the independence of the pump unit can be deactivated or disabled or at least restricted so that a given pump unit is operable with only one or a limited number of cassettes.

Generally, all terms used in the claims are to be interpreted according to their ordinary meaning in the technical field, unless explicitly defined otherwise herein. All references to a/an/the element, apparatus, member, component, means, etc. are to be interpreted openly as referring to at least one instance of the element, apparatus, member component, means, etc., unless explicitly stated otherwise.

### BRIEF DESCRIPTION OF THE DRAWINGS

Embodiments of the present disclosure will now be described by way of example only and with reference to the following accompanying drawings.
Figure 1A shows an embodiment of the present disclosure in a partially assembled state.
Figure 1B shows an embodiment of the present disclosure in an assembled state.
Figures 2A to 2C show different views of a pump unit according to an embodiment of the present disclosure.
Figures 3A to 3D show different views of a cassette according to an embodiment of the present disclosure.
Figure 4A shows a cross-sectional view of a cassette according to an embodiment of the present disclosure.
Figure 4B shows a cross-sectional view of a pump unit according to an embodiment of the present disclosure.
Figure 4C shows a cross-sectional view of a cassette attached to a pump unit according to an embodiment of the present disclosure.
Figure 5 shows a lower perspective view of a cassette attached to a pump unit according to an embodiment of the present disclosure.
Figures 6A to 6D show perspective views of different configurations of a system according to embodiments of the present disclosure.

### DETAILED DESCRIPTION

The working principle of the present disclosure will be briefly described. A more detailed description thereof is given below with reference to the drawings. The medicament delivery system as described herein comprises a cassette 1, wherein a flexible medicament bag 12 is provided in an interior of the cassette 1. The cassette 1 is air-tight except for an air inlet 15 in communication with an air outlet 32 of a pump unit 2 which can be attached to the cassette 1 (or vice versa). The pump unit 2 comprises an air pump 3 for pressurising the interior of the cassette 1 via the air outlet 32 and the air inlet 15. The air pressure acts on the outside of the medicament bag 12 and thus pushes the content of the medicament bag 12 through an outlet port 16 into an infusion set 5.

More than one cassette 1 is attachable and connectable with the pump unit 2. Each cassette 1 has its own air inlet 15 to enable pressurising of the cassette 1 and thereby injecting individual drugs. Moreover, each cassette 1 has an RFID tag or identification tag for storing data and, e.g., inform the pump unit 2 in which order the drugs shall be injected and at which rate. It may be handier for a patient receiving one multi-cassette assembly containing up to, e.g., four different cassettes 1. The cassettes are mounted together after they have been filled in a pharmacy by sliding them together to form an assembly, e.g., in a rectangular shape. The patient then merely has to attach and connect, for example insert, the pump unit 2 wherein said insertion movement automatically connects an air outlet 32 of the pump unit 2 to a respective air inlet 15 of the cassettes 1. The patient can, when receiving the multi-cassette inspect the drug visually. To facilitate visual inspection, the cassettes 1 may be (at least partially) made of a transparent material enabling full view of medicament bags 12 stored therein.

In the following, a pump unit 2 having four air outlets 32 and thus providing the possibility of connecting four cassettes 1 is shown. However, this is not to be construed as limiting and other numbers of cassettes 1 may be used with the pump unit 2 according to the present disclosure.

In general, the pump unit 2 may be reusable. The cassettes 1 may be provided as single-use devices or as refillable cassettes 1. The medicament bags 12 may have a volume of between 20 ml and 250 ml, preferably between 25 ml and 100 ml.

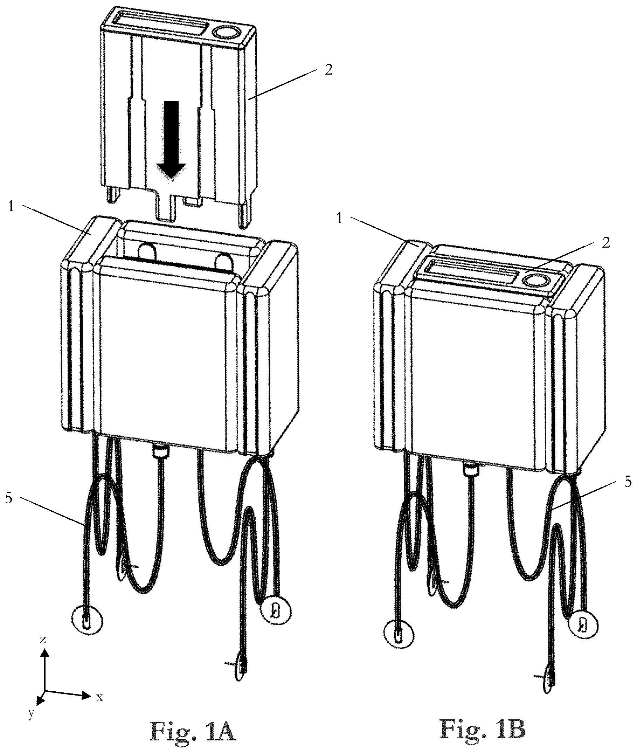

Fig. 1A shows a medicament delivery system according to an embodiment during assembly. The medicament delivery system comprises at least one cassette 1, in this case four cassettes 1, wherein infusion sets 5 are connected to each cassette 1, and a pump unit 2. The pump unit 2 is separably connected to the cassettes 1. In particular, the pump unit 2 can be attached to the cassettes 1 via a sliding motion as indicated by the arrow. In Fig. 1A, four cassettes 1 form a substantially rectangular shape, wherein the pump unit 2 is inserted in the middle of said rectangle in between the cassettes 1.

Fig. 1B shows the medicament delivery system in an assembled state. The pump unit 2 is in fluid connection to the cassettes 1 as will be described later and configured to pressurise an interior of at least one cassette 1. The system is ready for medicament delivery.

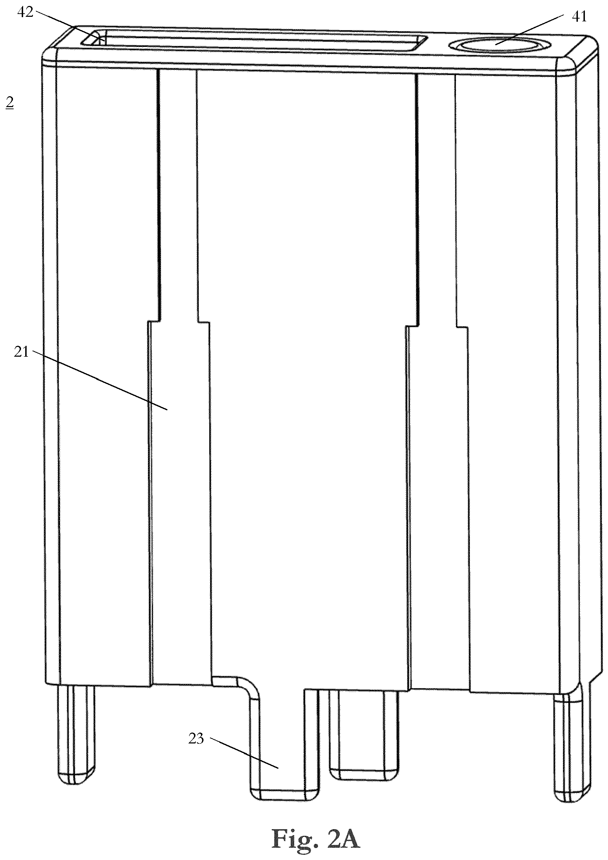

Fig. 2A shows a pump unit 2 according to an embodiment. The pump unit 2 may comprise an outer housing or shell. The housing may be formed of rigid plastics. The pump unit 2 may comprise at least one switch or button 41 for controlling medicament delivery and an indicator 42 comprising, e.g., a display or at least one light emitting element such as an LED and/or an audio signal generation unit such as a speaker. The switch 41 and/or the indicator 42 may be formed on an upper surface of the pump unit 2, i.e., the outer housing.

On at least one lateral side of the pump unit 2, at least one pump unit attachment means 21 may be formed. The term "means" as used herein may refer to a structural or physical feature provided at the pump unit 2 and/or the cassette 1. A "means" may, e.g., refer to a protrusion or a recess such as a guide rail or guide slot. "Attachment means" may thus denote a structural feature allowing attachment of one element to another.

The pump unit 2 may be substantially rectangular when viewed in the x-y-plane. The pump unit attachment means 21 may be formed on one of the longer sides of the pump unit 2. The pump unit attachment means 21 may extend in a vertical/longitudinal direction. In the figures, the attachment means 21 are formed as a longitudinally extending recess or slit. The attachment means 21 may be formed wider at a lower portion of the pump unit 2 to facilitate insertion and guidance of the corresponding cassette attachment means 13. The pump unit attachment means 21 may comprise an abutment or stop at a vertical end thereof, e.g., at a vertical end of the pump unit 2.

The pump unit 2 may further comprise tag protrusions 23 extending from a lower end of the pump unit 2. Inside the tag protrusion 23, an identification reader 22 or RFID reader to be described later may be provided.

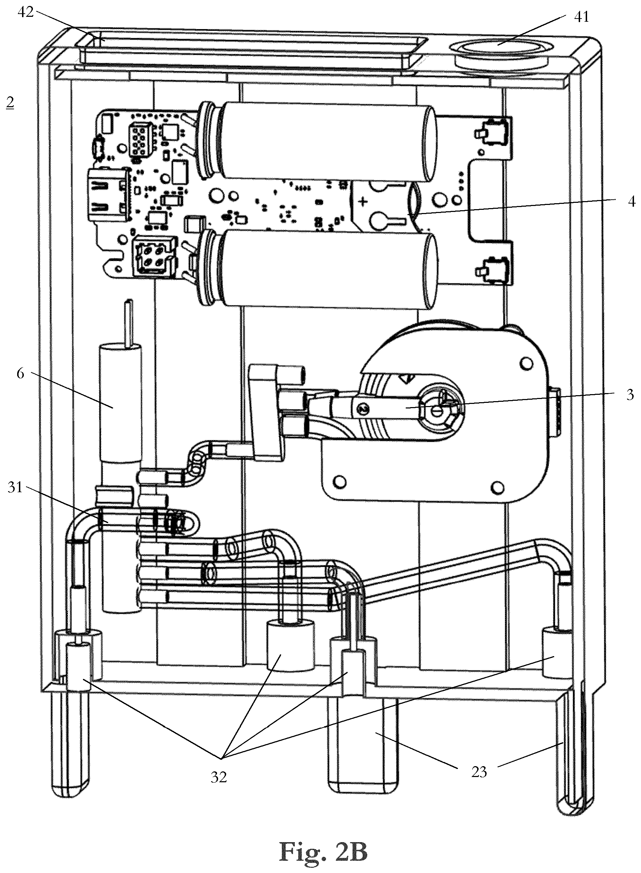

Fig. 2B shows an interior of a pump unit 2 according to an embodiment. Therein, a pump 3 is provided which is connected via tubing 31 to a valve 6 and at least one air outlet 32. In this case, four air outlets 32 are provided and the valve 6 is therefore a six-way-valve selectively providing communication between the ambient air, the pump 3, and at least one of the air outlets 32.

In general, an x-way valve may be provided, wherein x is the number of air outlets 32 plus two. In other words, x may be the maximum number of connectable cassettes 1 plus 2. In some embodiments, a plurality of air outlets 32 may be in fluid connection with the air pump 3 via the valve 6 at the same time.

The pump unit 2 may further comprise a processing unit 4. The processing unit 4 may comprise at least one of a memory, a processor, a power source, an interface (wireless/wired) and a sensor. The processing unit 4 may be configured to communicate with another device such as a mobile terminal (e.g., a smartphone), a computer, a cloud, a server or the internet via wired or wireless connection. The processing unit 4 may be configured to control at least one of the pump 3, the valve 6, or the indicator 42. The switch 41 may be connected to the processing unit 4 for controlling operation of the pump unit 2. The switch 41 may for example be provided to turn the pump unit 2 on or off and/or to start or stop medicament delivery.

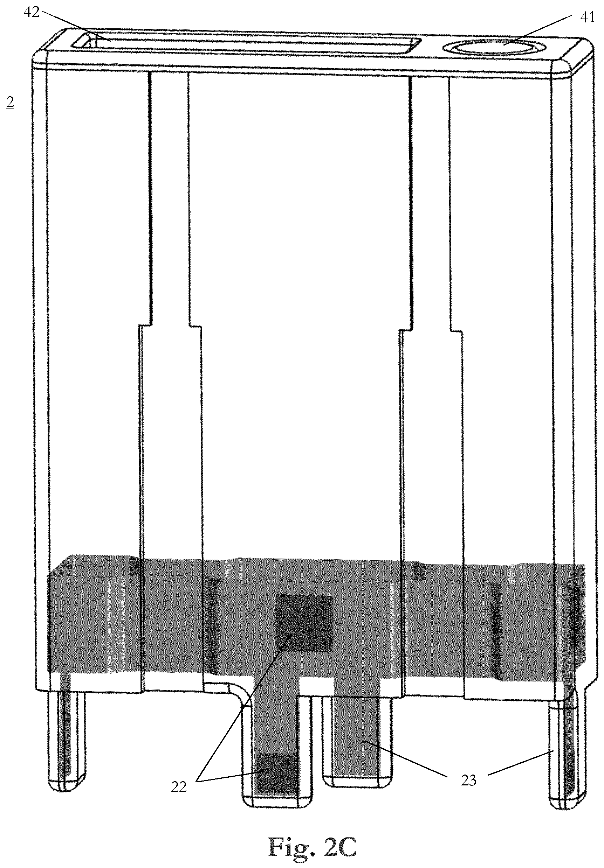

Fig. 2C shows a further perspective view of the pump unit 2, in which at least one identification reader 22 (RFID reader) is provided in the interior of the pump unit 2, i.e., the outer housing. The identification reader(s) 22 may be provided at positions corresponding to positions of identification tags such as RFID antennas in the cassette 1 and/or the infusion set 5. Thus, in the case of four cassettes 1 to be attached to a pump unit 2, and identification reader 22 may be provided each side of the pump unit 2. Preferably, the identification reader(s) 22 is/are provided at or towards a lower and of the pump unit 22. Consequently, each cassette 1 may comprise an identification tag at or towards a lower end of the cassette 1.

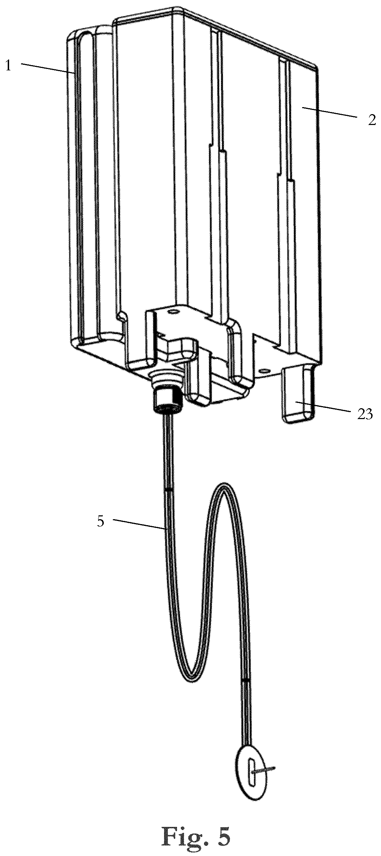

Moreover, each infusion set 5 may comprise an identification tag at the end that is connected to the cassette 1 (see Fig. 5). Hence, responding identification reader(s) 22 may be provided in the tag protrusions 23. The identification tags at the infusion sets 5 can be used to verify that an infusion set 5 and the correct infusion set 5 is attached to the cassette 1. Moreover, information of the correct needle penetration depth/infusion depth (i.e., subcutaneous or intramuscular), needle gauge, etc. may be provided.

The identification tags at the cassettes 1 may provide information on the drug contained in the medicament bag 12 such as the type of drug, volume of the drug, prescription parameters, required pressure for medicament delivery, injection rate, temperature, injection sequence/order of delivery (e.g., with respect to other cassettes 1/drugs), time of delivery, approved infusion set 5, expiry date, etc.

The at least one identification reader 22 may communicate with the processing unit 4. Thereby, it can be verified that the correct cassette(s) 1 is/are attached to the pump unit 2 and the medicaments are delivered according to the prescription. Thus, the processing unit 4 may control the valve 6 and the pump 3 to pressurise the cassette(s) 1 accordingly.

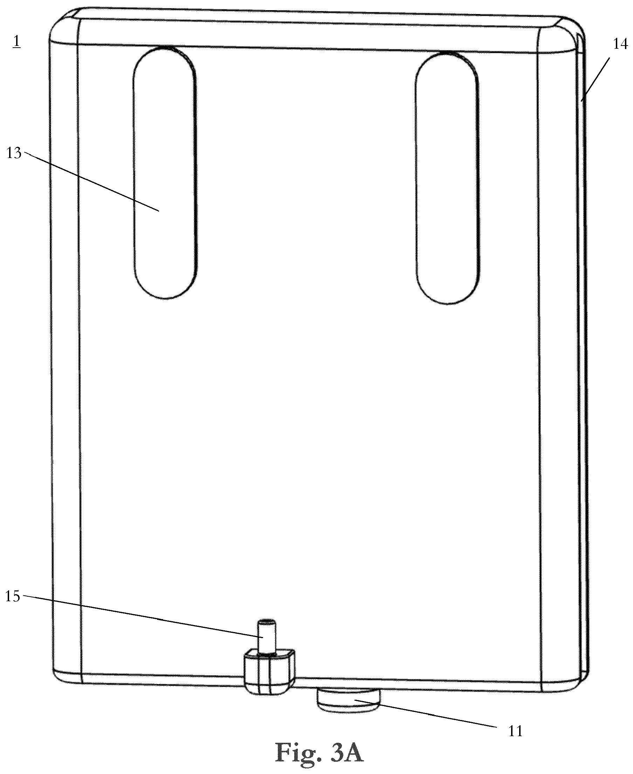

Fig. 3A shows a view of a cassette 1 according to an embodiment. The cassette 1 may comprise a hard outer shell or housing, and air inlet 15 and an opening 11. The cassette 1 may be hermetically sealed, i.e., air-tight, except for the air inlet 15 and the opening 11. An outlet port 16 of the medicament bag 12 may be provided in or protrude through the opening 11, wherein the opening 11 is also sealed in an air-tight manner when the outlet port 16 is inserted. The infusion set 5 may be connected to the outlet port 16.

The air inlet 15 may laterally protrude from the cassette 1. In particular, the air inlet 15 may have an L-shape. That is, the air inlet 15 may protrude in a lateral and vertical direction. The air inlet 15 may be disposed at or towards a bottom end of the cassette 1. The air inlet 15 may be provided at a longer side of the cassette 1.

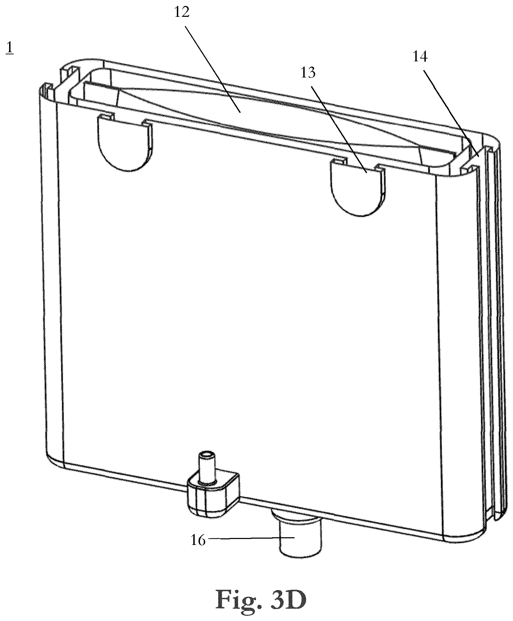

The cassette 1 further comprises cassette attachment means 13. The cassette attachment means 13 is configured to separably attach a pump unit 2 to the cassette 1 or vice versa. In the embodiment shown, the attachment means 13 is provided as two parallel longitudinally extending guide protrusions 13. The guide protrusions 13 may be provided at the longer side of the cassette 1. The guide protrusions 13 may be disposed at or towards an upper end of the cassette 1. The guide protrusions 13 may protrude in a transversal direction, i.e., normal to the longitudinal direction, and may be substantially T-shaped (cf. Fig. 3D).

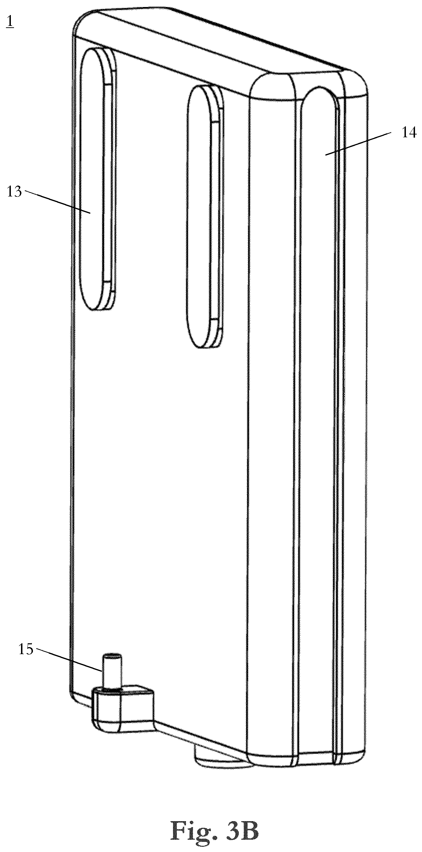

Referring to Fig. 3B, the cassette 1 may further comprise connection means or a connection interface 14. The connection interface 14 may be provided at a shorter side of the cassette 1 and may have a shape configured to receive the attachment means 13 of another cassette 1. That is, in Fig. 3B, the connection interface 14 is formed as a recess having a T-shape corresponding to the T -shaped guide protrusion 13. The connection interface 14 may extend in the longitudinal direction and may have an upper abutment or stop provided at or towards an upper end of the cassette 1.

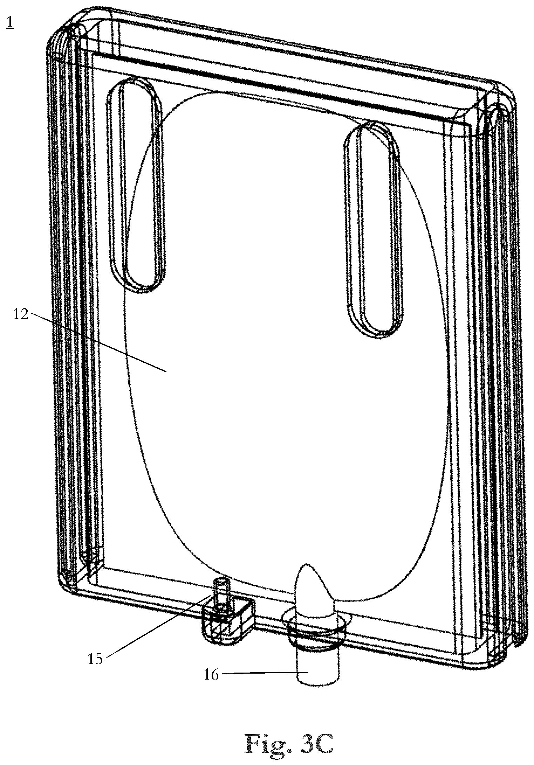

Fig. 3C shows a perspective view of a cassette 1, wherein the housing or the outer shell is transparently displayed. As can be seen from the figure, a flexible medicament bag 12 is provided in the interior of the cassette 1. The outlet port 16 of the medicament bag 12 is provided in or protrudes through the opening 11 such that an infusion set 5 (not shown) may be attached thereto. The outlet port 16 may be provided with a valve such as a pinch valve manually operable valve. For medicament delivery, the pressure inside the interior of the cassette 1 is increased by pumping air through the air inlet 15 as explained above. Since the cassette 1 is air-tight, the pressure acts on the flexible medicament bag 12 such that content of the medicament bag 12 is pushed through the outlet port 16.

Fig. 3D shows a cross-sectional view of a cassette 1 the x-y-plane. In this view, the medicament bag 12 and the space surrounding the medicament bag 12 which is pressurised for medicament delivery can be seen. Moreover, the exemplary T-shaped profile of the attachment means 13 and the connection interface 14 can be seen.

The attachment means 13, the connection interface 14 and the pump unit attachment means 21 may also be formed in a different shape allowing one or more cassettes 1 to be separately attached to a pump unit 2. In an embodiment, a plurality of cassettes 1 may be attached to each other and the pump unit 2 may be attached to the cassettes 1 by a sliding movement as shown in Fig. 1A. The profiles 13, 14, 21 may provide a tight fit in order to prevent the pump unit 2 or the cassette(s) 1 from accidentally separating from each other. The profiles 13, 14, 21 may also be provided contrary to the shown configuration. That is, the pump unit attachment means 21 may be formed as a (T-shaped) protrusion on the pump unit 2 and the cassette attachment means 13 may be formed as a corresponding (T-shaped) recess on the cassette 1. Consequently, the connection interface 14 may also be formed as a (T-shaped) protrusion.

Alternatively, the attachment means 13, 14, 21 may be alternately provided, i.e., comprising one protrusion and one recess.

The cassettes 1 may be provided in different volumes for different kinds of medication. The cassettes 1 may therefore differ in height and width as long as the positions of the cassette attachment means 13 and the connection means 14 allow attachment of a pump unit 2 and/or other cassettes 1.

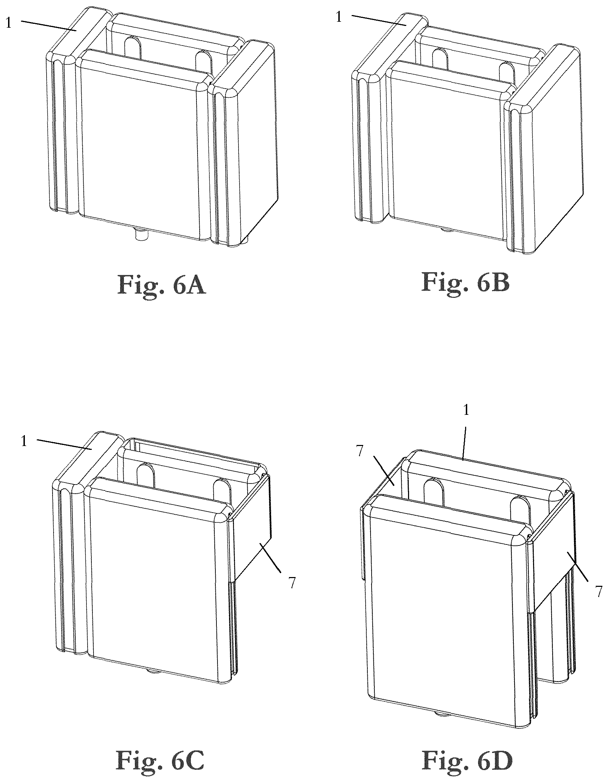

The medicament system disclosed herein may also comprise adapters 7 or dummy cassettes which are provided with the same cassette attachment means 13 (adapter attachment means 13) and connection means 14 (connection interface 14) as the cassettes 1. Thus, in case less than four medicaments are required, adapters 7 may be used to form a rectangular shape for insertion of a pump unit 2. The adapters 7 may be formed as empty cassettes (dummy cassettes) having connection means 13, 14 (i.e. adapter attachment means 13 and a connection interface 14 as the cassettes 1) or adapter plates 7 comprising cassette attachment means 13. The adapters 7 may be provided in longer or shorter versions depending on whether they connect cassettes 1 or other adapters 7 via the longer or the shorter side of the pump unit 2 (cf. Figs. 6A to 6D).

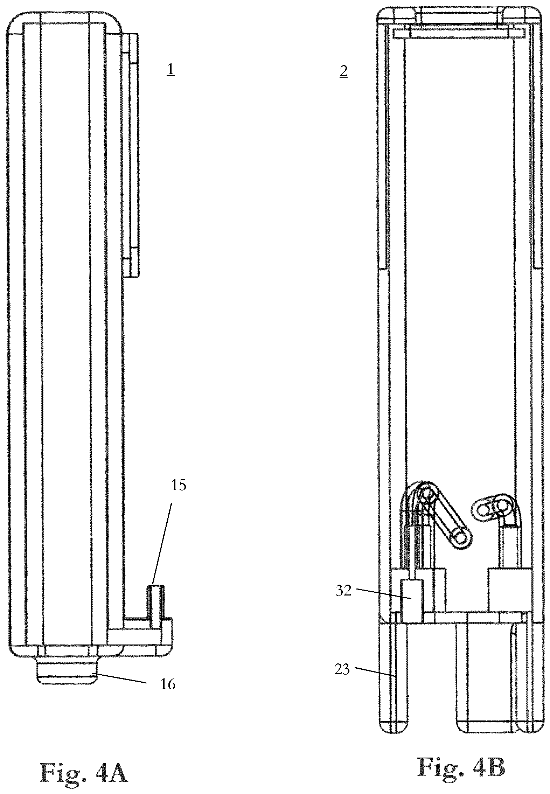

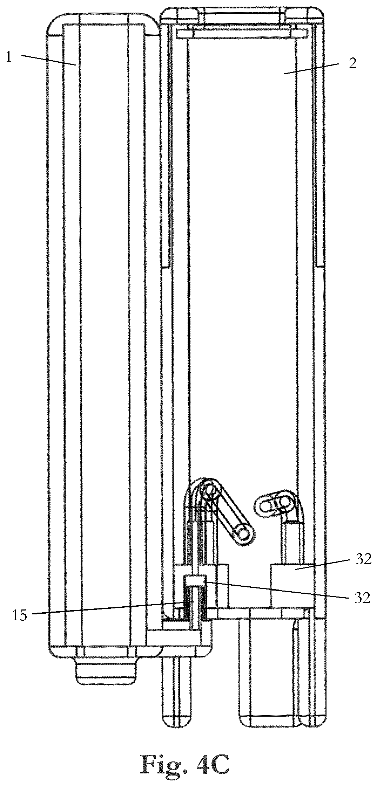

Fig. 4A shows a cross-sectional view of a cassette 1, Fig. 4B shows a cross-sectional view of a pump unit 2, and Fig. 4C shows a cross-sectional view of a pump unit 2 attached to a cassette 1. The laterally protruding part of the air outlet 15 may also form a lower stop or abutment for the pump unit 2. As can be seen from Fig. 4C, the air inlet 15 of the cassette 1 is in engagement with 1 of the air outlets 32 of the pump unit 2. Thus, a fluid connection between the air outlet 32 and the air inlet 15 is established. Said connection may be air-tight such that air pumped from the air pump 3 is not leaked to the ambient air but only used to pressurise the interior of the cassette 1.

Fig. 5 is a lower view of the medicament delivery system with one cassette 1 attached to a pump unit 2. As can be seen from the figure, the infusion set 5 may comprise an identification tag (RFID tag, indicated by the black surface), wherein the identification tag is placed at a position corresponding to one of the identification readers 22 provided in one of the tag protrusions 23.

The air inlet 15 may not be provided centrally at the respective sides of the cassette 1 but may be laterally offset with respect to a centre as shown in the figures. The air outlet 32 may thus be positioned respectively. This may allow the tag protrusions 23, if provided, to pass the air inlet 15 during insertion in a vertical direction. Consequently, the opening 11 may also be laterally offset with respect to a centre of the cassette 1.

Figs. 6A to 6D show different configurations of cassettes 1 and adapters 7 forming a substantially rectangular shape. In Fig. 6A, two larger cassettes 1 are provided at the longer side of the pump unit 2 (not shown) and two smaller cassettes 1 are connected to the larger cassettes 1 via their respective cassette attachment means 13 and connection interfaces 14. The cassettes 1 thus form a substantially rectangular shape, wherein the pump unit 2 can be inserted in the middle as described above.

Fig. 6B shows a configuration of four large cassettes 1 which are connected via their respective cassette attachment means 13 and connection interfaces 14 as described above.

In Fig. 6C, cassettes 1 of three different sizes and one adapter 7 are connected to form a substantially rectangular shape for insertion of a pump unit 2. In that configuration, the front cassette 1 is a large cassette, the left cassette 1 is a cassette having a smaller width than the large cassette and the back cassette 1 is a cassette having a smaller height than the large cassette. The front and back cassettes 1 are connected via an adapter 7 on the right.

According to Fig. 6D, two cassettes 1 are provided at the opposing longer sides of the pump unit 2 and connected via two adapters 7.

In an embodiment, the housing or outer shell of the cassette 1 may be formed of a transparent material. This allows visual inspection of the interior of the cassette 1 by a patient, pharmacist or physician. Moreover, the rectangular arrangement of cassettes 1, that is, the cassettes 1 surrounding the pump unit 2, further facilitates visual inspection of the cassettes 1 compared to, e.g., a stacked arrangement of cassettes 1.

The present disclosure also encompasses cassette 1, a pump unit 2 and an adapter 7 for use with the described medicament delivery system.

An exemplary medicament delivery may be performed as follows.

The patient may consult a healthcare professional (HCP) which describes a medication scheme consisting, e.g., of a combination of large volume subcutaneous injectable drugs with personalised dosing. The drugs may be injected in sequence or parallel according to the prescription. The HCP may store the drug prescription in a cloud-based system, a server, in a memory, or the like. A pharmacy may prepare the drugs, i.e., the cassettes 1 according to the prescription and hand them to the patient.

The pharmacy may receive or get access to the prescription through the cloud-based system, the server, the memory, etc. When preparing the cassettes 1, the pharmacy may add data regarding the grant, drug infusion sequence, dose, infusion rate, expiry date and patient data to the identification tag or RFID tag. Optionally, data of approved infusion sets 5 may also be stored on the identification tag.

The cassettes 1 may be delivered to the patient as an assembled unit, i.e., with all cassettes 1 and adapters 7, if necessary, attached to each other. Moreover, infusion sets 5 (with optional identification tags) may be delivered together with the assembled cassettes 1. Alternatively, the patient may assemble the cassettes 1 and adapters 7, if necessary, prior to medicament delivery.

The patient may visually inspect the drugs medicament bags 12 in the cassettes 1 before mounting the pump unit 2. Since the pump unit 2 is a reusable device, it may be provided to the patient separate from or together with the cassettes 1. Prior to medicament delivery, the patient connects all infusion sets 5 and mounts the needles to the body.

The pump unit 2 may identify each of the cassettes 1 and/or the infusion sets 5 using the identification readers 22 and the identification tags and process the data accordingly. The data may also be transmitted to a cloud, server or the like.

The pump unit 2 may verify that the patient, the cassettes 1 (and optionally the infusion sets 5) are correct and identifies the correct mounting position of the cassettes 1 so that the correct injection sequence is ensured.

For medicament delivery, the pump unit 2 injects the drugs according to the prescribed sequence by pressurising the cassettes 1. Therefore, the pump 3 sucks in ambient air and distributes it through the valve 6 to at least 1 of the cassettes 1. Injection speed may be controlled via the pressure and the pressure and/or the airflow may be monitored by sensors in the pump unit 2.

During medicament delivery, the indicator 42 may inform the patient of the injection progress. In particular, the indicator 42 may output audible and/or optical signals.

Moreover, during and/or after medicament delivery, data regarding the different states of the infusion may be transmitted to the cloud, the server, or the like.

The present disclosure also encompasses a pump unit 2 as described above for use with a system as described above. The present disclosure further encompasses a cassette 1 as described above for use with a system as described above.

The delivery devices described herein can be used for the treatment and/or prophylaxis of one or more of many different types of disorders.

Exemplary disorders include, but are not limited to: rheumatoid arthritis, inflammatory bowel diseases (e.g. Crohn's disease and ulcerative colitis), hypercholesterolaemia and/or dyslipidemia, cardiovascular disease, diabetes (e.g. type 1 or 2 diabetes), psoriasis, psoriatic arthritis, spondyloarthritis, hidradenitis suppurativa, Sjögren's syndrome, migraine, cluster headache, multiple sclerosis, neuromyelitis optica spectrum disorder, anaemia, thalassemia, paroxysmal nocturnal hemoglobinuria, hemolytic anaemia, hereditary angioedema, systemic lupus erythematosus, lupus nephritis, myasthenia gravis, Behçet's disease, hemophagocytic lymphohistiocytosis, atopic dermatitis, retinal diseases (e.g., age-related macular degeneration, diabetic macular edema), uveitis, infectious diseases, bone diseases (e.g., osteoporosis, osteopenia), asthma, chronic obstructive pulmonary disease, thyroid eye disease, nasal polyps, transplant, acute hypoglycaemia, obesity, anaphylaxis, allergies, sickle cell disease, Alzheimer's disease, Parkinson's disease, dementia with Lewy bodies, systemic infusion reactions, immunoglobulin E (IgE)-mediated hypersensitivity reactions, cytokine release syndrome, immune deficiencies (e.g., primary immunodeficiency, chronic inflammatory demyelinating polyneuropathy), enzyme deficiencies (e.g., Pompe disease, Fabry disease, Gaucher disease), growth factor deficiencies, hormone deficiencies, coagulation disorders (e.g., hemophilia, von Willebrand disease, Factor V Leiden), and cancer.

Exemplary types of drugs that could be included in the delivery devices described herein include, but are not limited to, small molecules, hormones, cytokines, blood products, enzymes, vaccines, anticoagulants, immunosuppressants, antibodies, antibody-drug conjugates, neutralizing antibodies, reversal agents, radioligand therapies, radioisotopes and/or nuclear medicines, diagnostic agents, bispecific antibodies, proteins, fusion proteins, peptibodies, polypeptides, pegylated proteins, protein fragments, nucleotides, protein analogues, protein variants, protein precursors, protein derivatives, chimeric antigen receptor T cell therapies, cell or gene therapies, oncolytic viruses, or immunotherapies.

Exemplary drugs that could be included in the delivery devices described herein include, but are not limited to, immuno-oncology or bio-oncology medications such as immune checkpoints, cytokines, chemokines, clusters of differentiation, interleukins, integrins, growth factors, coagulation factors, enzymes, enzyme inhibitors, retinoids, steroids, signaling proteins, pro-apoptotic proteins, anti-apoptotic proteins, T-cell receptors, B-cell receptors, or costimulatory proteins.

Exemplary drugs that could be included in the delivery devices described herein include, but are not limited to, those exhibiting a proposed mechanism of action, such as human epidermal growth factor receptor 2 (HER-2) receptor modulators, interleukin (IL) modulators, interferon (IFN) modulators, complement modulators, glucagon-like peptide-1 (GLP-1) modulators, glucose-dependent insulinotropic polypeptide (GIP) modulators, cluster of differentiation 38 (CD38) modulators, cluster of differentiation 22 (CD22) modulators, C1 esterase modulators, bradykinin modulators, C-C chemokine receptor type 4 (CCR4) modulators, vascular endothelial growth factor (VEGF) modulators, B-cell activating factor (BAFF), P-selectin modulators, neonatal Fc receptor (FcRn) modulators, calcitonin gene-related peptide (CGRP) modulators, epidermal growth factor receptor (EGFR) modulators, cluster of differentiation 79B (CD79B) modulators, tumor-associated calcium signal transducer 2 (Trop-2) modulators, cluster of differentiation 52 (CD52) modulators, B-cell maturation antigen (BCMA) modulators, enzyme modulators, platelet-derived growth factor receptor A (PDGFRA) modulators, cluster of differentiation 319 (CD319 or SLAMF7) modulators, programmed cell death protein 1 and programmed death-ligand 1 (PD-1/PD-L1) inhibitors/modulators, B-lymphocyte antigen cluster of differentiation 19 (CD19) inhibitors, B-lymphocyte antigen cluster of differentiation 20 (CD20) modulators, cluster of differentiation 3 (CD3) modulators, cytotoxic T-lymphocyte-associated protein 4 (CTLA-4) inhibitors, T-cell immunoglobulin and mucin-domain containing-3 (TIM-3) modulators, T cell immunoreceptor with Ig and ITIM domains (TIGIT) modulators, V-domain Ig suppressor of T cell activation (VISTA) modulators, indoleamine 2,3-dioxygenase (IDO or INDO) modulators, poliovirus receptor-related immunoglobulin domain-containing protein (PVRIG) modulators, lymphocyte-activation gene 3 (LAG3; also known as cluster of differentiation 223 or CD223) antagonists, cluster of differentiation 276 (CD276 or B7-H3) antigen modulators, cluster of differentiation 47 (CD47) antagonists, cluster of differentiation 30 (CD30) modulators, cluster of differentiation 73 (CD73) modulators, cluster of differentiation 66 (CD66) modulators, cluster of differentiation w137 (CDw137) agonists, cluster of differentiation 158 (CD158) modulators, cluster of differentiation 27 (CD27) modulators, cluster of differentiation 58 (CD58) modulators, cluster of differentiation 80 (CD80) modulators, cluster of differentiation 33 (CD33) modulators, cluster of differentiation 159 (CD159 or NKG2) modulators, glucocorticoid-induced TNFR-related (GITR) protein modulators, Killer Ig-like receptor (KIR) modulators, growth arrest-specific protein 6 (GAS6)/AXL pathway modulators, A proliferation-inducing ligand (APRIL) receptor modulators, human leukocyte antigen (HLA) modulators, epidermal growth factor receptor (EGFR) modulators, B-lymphocyte cell adhesion molecule modulators, cluster of differentiation w123 (CDw123) modulators, Erbb2 tyrosine kinase receptor modulators, endoglin modulators, mucin modulators, mesothelin modulators, hepatitis A virus cellular receptor 2 (HAVCR2) antagonists, cancer-testis antigen (CTA) modulators, tumor necrosis factor receptor superfamily, member 4 (TNFRSF4 or OX40) modulators, adenosine receptor modulators, inducible T cell co-stimulator (ICOS) modulators, cluster of differentiation 40 (CD40) modulators, tumor-infiltrating lymphocytes (TIL) therapies, or T-cell receptor (TCR) therapies.

Exemplary drugs that could be included in the delivery devices described herein include, but are not limited to: etanercept, abatacept, adalimumab, evolocumab, exenatide, secukinumab, erenumab, galcanezumab, fremanezumab-vfrm, alirocumab, methotrexate (amethopterin), tocilizumab, interferon beta-1a, interferon beta-1b, peginterferon beta-1a, sumatriptan, darbepoetin alfa, belimumab, sarilumab, semaglutide, dupilumab, reslizumab, omalizumab, glucagon, epinephrine, naloxone, insulin, amylin, vedolizumab, eculizumab, ravulizumab, crizanlizumab-tmca, certolizumab pegol, satralizumab, denosumab, romosozumab, benralizumab, emicizumab, tildrakizumab, ocrelizumab, ofatumumab, natalizumab, mepolizumab, risankizumab-rzaa, ixekizumab, and immune globulins.

Exemplary drugs that could be included in the delivery devices described herein may also include, but are not limited to, oncology treatments such as ipilimumab, nivolumab, pembrolizumab, atezolizumab, durvalumab, avelumab, cemiplimab, rituximab, trastuzumab, ado-trastuzumab emtansine, fam-trastuzumab deruxtecan-nxki, pertuzumab, transtuzumab-pertuzumab, alemtuzumab, belantamab mafodotin-blmf, bevacizumab, blinatumomab, brentuximab vedotin, cetuximab, daratumumab, elotuzumab, gemtuzumab ozogamicin, 90-Yttrium-ibritumomab tiuxetan, isatuximab, mogamulizumab, moxetumomab pasudotox, obinutuzumab, ofatumumab, olaratumab, panitumumab, polatuzumab vedotin, ramucirumab, sacituzumab govitecan, tafasitamab, or margetuximab.

Exemplary drugs that could be included in the delivery devices described herein include "generic" or biosimilar equivalents of any of the foregoing, and the foregoing molecular names should not be construed as limiting to the "innovator" or "branded" version of each, as in the non-limiting example of innovator medicament adalimumab and biosimilars such as adalimumab-afzb, adalimumab-atto, adalimumab-adbm, and adalimumab-adaz.

Exemplary drugs that could be included in the delivery devices described herein also include, but are not limited to, those used for adjuvant or neoadjuvant chemotherapy, such as an alkylating agent, plant alkaloid, antitumor antibiotic, antimetabolite, or topoisomerase inhibitor, enzyme, retinoid, or corticosteroid. Exemplary chemotherapy drugs include, by way of example but not limitation, 5-fluorouracil, cisplatin, carboplatin, oxaliplatin, doxorubicin, daunorubicin, idarubicin, epirubicin, paclitaxel, docetaxel, cyclophosphamide, ifosfamide, azacitidine, decitabine, bendamustine, bleomycin, bortezomib, busulfan, cabazitaxel, carmustine, cladribine, cytarabine, dacarbazine, etoposide, fludarabine, gemcitabine, irinotecan, leucovorin, melphalan, methotrexate, pemetrexed, mitomycin, mitoxantrone, temsirolimus, topotecan, valrubicin, vincristine, vinblastine, or vinorelbine.

Exemplary drugs that could be included in the delivery devices described herein also include, but are not limited to, analgesics (e.g., acetaminophen), antipyretics, corticosteroids (e.g. hydrocortisone, dexamethasone, or methylprednisolone), antihistamines (e.g., diphenhydramine or famotidine), antiemetics (e.g., ondansetron), antibiotics, antiseptics, anticoagulants, fibrinolytics (e.g., recombinant tissue plasminogen activator [r-TPA]), antithrombolytics, or diluents such as sterile water for injection (SWFI), 0.9% Normal Saline, 0.45% normal saline, 5% dextrose in water, 5% dextrose in 0.45% normal saline, Lactated Ringer's solution, Heparin Lock Flush solution, 100 U/mL Heparin Lock Flush Solution, or 5000 U/mL Heparin Lock Flush Solution.

Pharmaceutical formulations including, but not limited to, any drug described herein are also contemplated for use in the delivery devices described herein, for example pharmaceutical formulations comprising a drug as listed herein (or a pharmaceutically acceptable salt of the drug) and a pharmaceutically acceptable carrier. Such formulations may include one or more other active ingredients (e.g., as a combination of one or more active drugs), or may be the only active ingredient present, and may also include separately administered or co-formulated dispersion enhancers (e.g. an animal-derived, human-derived, or recombinant hyaluronidase enzyme), concentration modifiers or enhancers, stabilizers, buffers, or other excipients.

Exemplary drugs that could be included in the delivery devices described herein include, but are not limited to, a multi-medication treatment regimen such as AC, Dose-Dense AC, TCH, GT, EC, TAC, TC, TCHP, CMF, FOLFOX, mFOLFOX6, mFOLFOX7, FOLFCIS, CapeOx, FLOT, DCF, FOLFIRI, FOLFIRINOX, FOLFOXIRI, IROX, CHOP, R-CHOP, RCHOP-21, Mini-CHOP, Maxi-CHOP, VR-CAP, Dose-Dense CHOP, EPOCH, Dose-Adjusted EPOCH, R-EPOCH, CODOX-M, IVAC, HyperCVAD, R-HyperCVAD, SC-EPOCH-RR, DHAP, ESHAP, GDP, ICE, MINE, CEPP, CDOP, GemOx, CEOP, CEPP, CHOEP, CHP, GCVP, DHAX, CALGB 8811, HIDAC, MOpAD, 7 + 3, 5 +2, 7 + 4, MEC, CVP, RBAC500, DHA-Cis, DHA-Ca, DHA-Ox, RCVP, RCEPP, RCEOP, CMV, DDMVAC, GemFLP, ITP, VIDE, VDC, VAI, VDC-IE, MAP, PCV, FCR, FR, PCR, HDMP, OFAR, EMA/CO, EMA/EP, EP/EMA, TP/TE, BEP, TIP, VIP, TPEx, ABVD, BEACOPP, AVD, Mini-BEAM, IGEV, C-MOPP, GCD, GEMOX, CAV, DT-PACE, VTD-PACE, DCEP, ATG, VAC, VeIP, OFF, GTX, CAV, AD, MAID, AIM, VAC-IE, ADOC, or PE.

Various modifications to the embodiments described are possible and will occur to those skilled in the art without departing from the invention which is defined by the following claims.

## Claims

1. Medicament delivery system comprising
at least one cassette (1), wherein the cassette (1) comprises a medicament bag (12) provided in an interior of the cassette (1) and an air inlet (15) in fluid communication with the interior of the cassette (1),
a pump unit (2), wherein the pump unit (2) comprises an air pump (3), a valve (6) and an air outlet (32),
wherein each cassette (1) comprises cassette attachment means (13), and the pump unit (2) comprises corresponding pump unit attachment means (21), wherein the pump unit (2) is configured to be separably attached to an outer circumference of at least one cassette (1),
wherein the air outlet (32) and the air inlet (15) are fluidly connected after attachment of the at least one cassette (1) with the pump unit (2), and
wherein the air pump (3) is configured to pressurise the interior of the cassette (1) via the connected air outlet (32) and the air inlet (15),
**characterized in that** the at least one cassette (1) comprises a connection interface (14) configured to be engageable with the cassette attachment means (13) of another cassette.

2. Medicament delivery system according to claim 1, wherein the valve (6) is configured to selectively direct an air flow from the pump (3) to one of the at least one cassettes (1).

3. Medicament delivery system according to any one of claims 1 or 2, wherein the at least one cassette (1) comprises at least one guide protrusion as attachment means (13) and the pump unit (2) comprises at least one corresponding guide recess as attachment means (21), or
wherein the pump unit (2) comprises at least one guide protrusion as attachment means (21) and the at least one cassette (1) comprises at least one corresponding guide recess as attachment means (13),
wherein the at least one cassette (1) is separably attached to the pump unit (2) via the guide protrusion (13, 21) and the guide recess (13, 21).

4. Medicament delivery system according to any one of claims 1 to 3, wherein the system comprises four cassettes (1) including the at least one cassette that are attached to each other by the connection interface (14) and the cassette attachment means (13), wherein the four cassettes (1) are configured to be arranged in a 90-degree angle with respect to each other or in a substantially rectangular shape, and wherein the cassettes are configured to be connected to each other such that they surround the pump unit (2).

5. Medicament delivery system according to any one of claims 1 to 4, wherein the system further comprises at least one adapter (7) comprising cassette attachment means (13) configured to engage the connection interface (14) of at least one cassette (1) or the pump unit attachment means (21), wherein the at least one adapter is formed as an empty cassette or a plate.

6. Medicament delivery system according to any one of claims 1 to 3, wherein the system comprises one or more further cassettes and/or one or more adapters (7) formed as an empty cassette or a plate, wherein the at least one cassette (1) and one or more further cassettes and/or one or more adapters (7) are configured to be connected to each other such that they surround the pump unit (2).

7. Medicament delivery system according to any one of claims 1 to 6, wherein the valve (6) is an x-way valve, wherein x equals the number of air outlets (32) plus two.

8. Medicament delivery system according to any one of claims 1 to 7, wherein the air inlet (15) is disposed at a lower end of the cassette (1) and the air outlet (32) is disposed at a lower end of the pump unit (2).

9. Medicament delivery system according to any one of claims 1 to 8, wherein the air outlet (32) and/or the air inlet (15) comprises a sealing member configured to seal the connection between the air outlet (32) and the air inlet (15) in an air-tight manner when the at least one cassette (1) is attached to the pump unit (2).

10. Medicament delivery system according to any one of claims 1 to 9, wherein an opening (11) is provided in a bottom surface of the cassette (1) and an outlet port (16) of the medicament bag (12) is provided in the opening (11).

11. Medicament delivery system according to any one of claims 1 to 10, wherein the pump unit (2) comprises a switch (41) and/or an indicator (42), wherein the switch (41) and/or indicator (42) is/are provided in a top surface opposite the bottom surface.

12. Medicament delivery system according to any one of claims 1 to 11, wherein the at least one cassette (1) comprises an identification tag, and
wherein the pump unit (2) comprises at least one identification reader (22) configured to read the identification tag.

13. Medicament delivery system according to any one of claims 1 to 12, wherein the system further comprises an infusion set (5) for each cassette (1) and each medicament bag (12) comprises an outlet port (16) configured to receive the infusion set (5).

14. Medicament delivery system according to any one of claims 1 to 13, wherein the pump unit (2) comprises a processing unit (4) configured to control at least one of the air pump (3), the valve (6), the indicator (42) or the identification antenna, wherein preferably, the switch (41) is connected to the processing unit (4).

## Patentansprüche

1. Arzneimittelabgabesystem, umfassend
mindestens eine Kassette (1), wobei die Kassette (1) einen Arzneimittelbeutel (12), der in einem Inneren der Kassette (1) bereitgestellt wird, und einen Lufteinlass (15) in Fluidkommunikation mit dem Inneren der Kassette (1) umfasst,
eine Pumpeneinheit (2), wobei die Pumpeneinheit (2) eine Luftpumpe (3), ein Ventil (6) und einen Luftauslass (32) umfasst,
wobei jede Kassette (1) Kassettenbefestigungsmittel (13) umfasst, und die Pumpeneinheit (2) entsprechende Pumpeneinheitsbefestigungsmittel (21) umfasst, wobei die Pumpeneinheit (2) konfiguriert ist, um an einem Außenumfang mindestens einer Kassette (1) trennbar befestigt zu werden,
wobei der Luftauslass (32) und der Lufteinlass (15) nach einer Befestigung der mindestens einen Kassette (1) mit der Pumpeinheit (2) fluidisch verbunden sind, und
wobei die Luftpumpe (3) konfiguriert ist, um das Innere der Kassette (1) über den verbundenen Luftauslass (32) und den Lufteinlass (15) unter Druck zu setzen,
**dadurch gekennzeichnet, dass** die mindestens eine Kassette (1) eine Verbindungsschnittstelle (14) umfasst, die konfiguriert ist, um mit den Kassettenbefestigungsmitteln (13) einer anderen Kassette in Eingriff bringbar zu sein.

2. Arzneimittelabgabesystem nach Anspruch 1, wobei das Ventil (6) konfiguriert ist, um einen Luftstrom von der Pumpe (3) selektiv zu einer der mindestens einen Kassetten (1) zu leiten.

3. Arzneimittelabgabesystem nach einem der Ansprüche 1 oder 2, wobei die mindestens eine Kassette (1) mindestens einen Führungsvorsprung als Befestigungsmittel (13) umfasst und die Pumpeneinheit (2) mindestens eine entsprechende Führungsausnehmung als Befestigungsmittel (21) umfasst, oder
wobei die Pumpeneinheit (2) mindestens einen Führungsvorsprung als Befestigungsmittel (21) umfasst und die mindestens eine Kassette (1) mindestens eine entsprechende Führungsaussparung als Befestigungsmittel (13) umfasst,
wobei die mindestens eine Kassette (1) über den Führungsvorsprung (13, 21) und die Führungsausnehmung (13, 21) an der Pumpeinheit (2) trennbar befestigt ist.

4. Arzneimittelabgabesystem nach einem der Ansprüche 1 bis 3, wobei das System vier Kassetten (1) umfasst, die die mindestens eine Kassette einschließen, die durch die Verbindungsschnittstelle (14) und die Kassettenbefestigungsmittel (13) aneinander befestigt sind, wobei die vier Kassetten (1) konfiguriert sind, um in einem 90-Grad-Winkel hinsichtlich zueinander oder in einer im Wesentlichen Rechteckform angeordnet zu sein, und wobei die Kassetten konfiguriert sind, um derart miteinander verbunden zu werden, dass sie die Pumpeneinheit (2) umgeben.

5. Arzneimittelabgabesystem nach einem der Ansprüche 1 bis 4, wobei das System ferner mindestens einen Adapter (7) umfasst, umfassend Kassettenbefestigungsmittel (13), die konfiguriert sind, um mit der Verbindungsschnittstelle (14) mindestens einer Kassette (1) oder den Pumpeneinheitsbefestigungsmitteln (21) in Eingriff zu stehen, wobei der mindestens eine Adapter als eine leere Kassette oder eine Platte ausgebildet ist.

6. Arzneimittelabgabesystem nach einem der Ansprüche 1 bis 3, wobei das System eine oder mehrere weitere Kassetten und/oder einen oder mehrere Adapter (7) umfasst, die als eine leere Kassette oder eine Platte ausgebildet sind, wobei die mindestens eine Kassette (1) und eine oder mehrere weitere Kassetten und/oder ein oder mehrere Adapter (7) konfiguriert sind, um derart miteinander verbunden zu werden, dass sie die Pumpeneinheit (2) umgeben.

7. Arzneimittelabgabesystem nach einem der Ansprüche 1 bis 6, wobei das Ventil (6) ein x-Wege-Ventil ist, wobei x gleich der Anzahl der Luftauslässe (32) plus zwei ist.

8. Arzneimittelabgabesystem nach einem der Ansprüche 1 bis 7, wobei der Lufteinlass (15) an einem unteren Ende der Kassette (1) eingerichtet ist und der Luftauslass (32) an einem unteren Ende der Pumpeinheit (2) eingerichtet ist.

9. Arzneimittelabgabesystem nach einem der Ansprüche 1 bis 8, wobei der Luftauslass (32) und/oder der Lufteinlass (15) ein Dichtungselement umfasst, das konfiguriert, um die Verbindung zwischen dem Luftauslass (32) und dem Lufteinlass (15) auf eine luftdichte Weise abzudichten, wenn die mindestens eine Kassette (1) an der Pumpeinheit (2) befestigt ist.

10. Arzneimittelabgabesystem nach einem der Ansprüche 1 bis 9, wobei eine Öffnung (11) in einer unteren Oberfläche der Kassette (1) bereitgestellt ist und ein Auslassanschluss (16) des Arzneimittelbeutels (12) in der Öffnung (11) bereitgestellt ist.

11. Arzneimittelabgabesystem nach einem der Ansprüche 1 bis 10, wobei die Pumpeneinheit (2) einen Schalter (41) und/oder einen Indikator (42) umfasst, wobei der Schalter (41) und/oder der Indikator (42) in einer oberen Oberfläche gegenüber der unteren Oberfläche bereitgestellt ist/sind.

12. Arzneimittelabgabesystem nach einem der Ansprüche 1 bis 11, wobei die mindestens eine Kassette (1) ein Identifikationsetikett umfasst, und
wobei die Pumpeneinheit (2) mindestens einen Identifikationsleser (22) umfasst, der konfiguriert ist, um das Identifikationsetikett zu lesen.

13. Arzneimittelabgabesystem nach einem der Ansprüche 1 bis 12, wobei das System ferner ein Infusionsset (5) für jede Kassette (1) umfasst und jeder Arzneimittelbeutel (12) einen Auslassanschluss (16) umfasst, der konfiguriert ist, um das Infusionsset (5) aufzunehmen.

14. Arzneimittelabgabesystem nach einem der Ansprüche 1 bis 13, wobei die Pumpeneinheit (2) eine Verarbeitungseinheit (4) umfasst, die konfiguriert ist, um mindestens eines von der Luftpumpe (3), dem Ventil (6), dem Indikator (42) oder der Identifikationsantenne zu steuern, wobei vorzugsweise der Schalter (41) mit der Verarbeitungseinheit (4) verbunden ist.

## Revendications

1. Système de délivrance de médicament comprenant
au moins une cassette (1), dans lequel la cassette (1) comprend une poche de médicament (12) fournie dans un intérieur de la cassette (1) et une entrée d'air (15) en communication fluidique avec l'intérieur de la cassette (1),
une unité de pompe (2), dans lequel l'unité de pompe (2) comprend une pompe à air (3), une valve (6) et une sortie d'air (32),
dans lequel chaque cassette (1) comprend un moyen de fixation de cassette (13), et l'unité de pompe (2) comprend un moyen de fixation d'unité de pompe (21) correspondant, dans lequel l'unité de pompe (2) est conçue pour être fixée de manière séparable à une circonférence externe d'au moins une cassette (1),
dans lequel la sortie d'air (32) et l'entrée d'air (15) sont raccordées fluidiquement après fixation de l'au moins une cassette (1) avec l'unité de pompe (2), et
dans lequel la pompe à air (3) est conçue pour pressuriser l'intérieur de la cassette (1) par l'intermédiaire de la sortie d'air (32) raccordée et de l'entrée d'air (15),
**caractérisé en ce que** l'au moins une cassette (1) comprend une interface de raccordement (14) conçue pour pouvoir venir en prise avec le moyen de fixation de cassette (13) d'une autre cassette.

2. Système de délivrance de médicament selon la revendication 1, dans lequel la valve (6) est conçue pour diriger sélectivement un flux d'air provenant de la pompe (3) vers l'une parmi l'au moins une cassette (1).

3. Système de délivrance de médicament selon l'une quelconque des revendications 1 ou 2, dans lequel l'au moins une cassette (1) comprend au moins une saillie de guidage en guise de moyen de fixation (13) et l'unité de pompe (2) comprend au moins un évidement de guidage correspondant en guise de moyen de fixation (21), ou
dans lequel l'unité de pompe (2) comprend au moins une saillie de guidage en guise de moyen de fixation (21) et l'au moins une cassette (1) comprend au moins un évidement de guidage correspondant en guise de moyen de fixation (13),
dans lequel l'au moins une cassette (1) est fixée de manière séparable à l'unité de pompe (2) par l'intermédiaire de la saillie de guidage (13, 21) et de l'évidement de guidage (13, 21).

4. Système de délivrance de médicament selon l'une quelconque des revendications 1 à 3, dans lequel le système comprend quatre cassettes (1) y compris l'au moins une cassette, qui sont fixées l'une à l'autre par l'interface de raccordement (14) et le moyen de fixation de cassette (13), dans lequel les quatre cassettes (1) sont conçues pour être agencées à un angle de 90 degrés les unes par rapport aux autres ou dans une forme sensiblement rectangulaire, et dans lequel les cassettes sont conçues pour être raccordées les unes aux autres de telle sorte qu'elles entourent l'unité de pompe (2).

5. Système de délivrance de médicament selon l'une quelconque des revendications 1 à 4, dans lequel le système comprend en outre au moins un adaptateur (7) comprenant un moyen de fixation de cassette (13) conçu pour venir en prise avec l'interface de raccordement (14) d'au moins une cassette (1) ou le moyen de fixation d'unité de pompe (21), dans lequel l'au moins un adaptateur est formé en tant que cassette vide ou en tant que plaque.

6. Système de délivrance de médicament selon l'une quelconque des revendications 1 à 3, dans lequel le système comprend une ou plusieurs cassettes supplémentaires et/ou un ou plusieurs adaptateurs (7) formés en tant que cassette vide ou en tant que plaque, dans lequel l'au moins une cassette (1) et une ou plusieurs cassettes supplémentaires et/ou un ou plusieurs adaptateurs (7) sont conçus pour être raccordés les uns aux autres de telle sorte qu'ils entourent l'unité de pompe (2).

7. Système de délivrance de médicament selon l'une quelconque des revendications 1 à 6, dans lequel la valve (6) est une valve à x voies, dans lequel x est égal au nombre de sorties d'air (32) plus deux.

8. Système de délivrance de médicament selon l'une quelconque des revendications 1 à 7, dans lequel l'entrée d'air (15) est disposée au niveau d'une extrémité inférieure de la cassette (1) et la sortie d'air (32) est disposée au niveau d'une extrémité inférieure de l'unité de pompe (2).

9. Système de délivrance de médicament selon l'une quelconque des revendications 1 à 8, dans lequel la sortie d'air (32) et/ou l'entrée d'air (15) comprennent un élément d'étanchéité conçu pour assurer l'étanchéité du raccordement entre la sortie d'air (32) et l'entrée d'air (15) d'une manière étanche à l'air lorsque l'au moins une cassette (1) est fixée à l'unité de pompe (2).

10. Système de délivrance de médicament selon l'une quelconque des revendications 1 à 9, dans lequel une ouverture (11) est fournie dans une surface inférieure de la cassette (1) et un orifice de sortie (16) de la poche de médicament (12) est fourni dans l'ouverture (11).

11. Système de délivrance de médicament selon l'une quelconque des revendications 1 à 10, dans lequel l'unité de pompe (2) comprend un commutateur (41) et/ou un indicateur (42), dans lequel le commutateur (41) et/ou l'indicateur (42) est/sont fourni(s) dans une surface supérieure opposée à la surface inférieure.

12. Système de délivrance de médicament selon l'une quelconque des revendications 1 à 11, dans lequel l'au moins une cassette (1) comprend une étiquette d'identification, et
dans lequel l'unité de pompe (2) comprend au moins un lecteur d'identification (22) configuré pour lire l'étiquette d'identification.

13. Système de délivrance de médicament selon l'une quelconque des revendications 1 à 12, dans lequel le système comprend en outre un ensemble à perfusion (5) pour chaque cassette (1) et chaque poche de médicament (12) comprend un orifice de sortie (16) conçu pour recevoir l'ensemble à perfusion (5).

14. Système de délivrance de médicament selon l'une quelconque des revendications 1 à 13, dans lequel l'unité de pompe (2) comprend une unité de traitement (4) configurée pour commander au moins l'un parmi la pompe à air (3), la valve (6), l'indicateur (42) ou l'antenne d'identification, dans lequel de préférence, le commutateur (41) est connecté à l'unité de traitement (4).
